# EUROPÄISCHE PATENTSCHRIFT

(11) **EP 0 763 370 B1**
(45) Veröffentlichungstag und Bekanntmachung des Hinweises auf die Patenterteilung: **22.08.2001**
(21) Anmeldenummer: 96118183.1
(22) Anmeldetag: 13.10.1993
(51) Int. Cl.: A61M 25/06, A61B 17/34

(54) **Katheterisierungsbesteck**
Catheterisation set
Ensemble de cathétèrisme

(30) Priorität: 24.11.1992 DE 9215927 U
(43) Veröffentlichungstag der Anmeldung: 19.03.1997
(62) Teilanmeldung aus: 93922943.1
(73) Patentinhaber: B. BRAUN MELSUNGEN AG, 34212 Melsungen (DE)
(72) Erfinder: Jesch, Franz, Dr., 82152 Krailling (DE)
(74) Vertreter: Selting, Günther, Dipl.-Ing.

(56) Entgegenhaltungen:
- EP-A- 0 129 745
- EP-A- 0 161 636
- EP-A- 0 352 928
- EP-A- 0 411 605
- WO-A-88/07388
- WO-A-92/18193
- WO-A-93/11812
- DE-A- 2 052 364
- DE-U- 8 914 941
- DE-U- 8 915 299
- FR-A- 2 368 968
- US-A- 3 680 562
- US-A- 4 191 186

## Beschreibung

Die Erfindung bezieht sich auf ein Katheterisierungsbesteck für die Katheterverlegung in einem Blutgefäß, bestehend aus einer Punktionskanüle mit einem Kanülenansatz, der eine Griffvorrichtung aufweist, einem die Punktionskanüle umgebenden Katheter mit einem Katheteransatz, und einem Führungsdraht, der in dem Lumen der Punktionskanüle axial verschiebbar ist.

Zur Messung von Drücken in Gefäßen (arterieller Blutdruck) sowie zur Verabreichung von Infusionen und Blutabnahmen müssen Arterien und zentrale Venen punktiert und sogenannte Verweilkanülen plaziert werden, die als kurze Katheter mit einem Katheteransatz gestaltet sind, an den eine Flüssigkeitsübertragungsvorrichtung anschließbar ist. In dem Katheter steckt eine Punktionskanüle, deren angeschärfte Spitze über das vordere Ende des Katheters etwas vorsteht. Ein solches Besteck ist bekannt aus US-A-3 312 220. Damit bei der Punktion der Übergang von der Punktionskanüle auf den dickeren Katheter allmählich erfolgt, ist die Spitze des Katheters außen nach vorne abgeschrägt. Der stufenlose Anschluß des Katheters an den Außenumfang der Punktionskanüle vermag jedoch nicht die Nachteile der Punktionslochaufweitung und der Erschwerung der Durchdringung der Haut und Gefäßwand zu vermeiden, die die Präzision der Punktion verringern. Letzteres wird dadurch verstärkt, daß bei diesem Besteck eine Griffvorrichtung fehlt, so daß es direkt am zylindrischen Kanülenansatz angefaßt werden muß und hier nur unsicher festgehalten werden kann.

Häufig ist es günstig, zur Plazierung des Katheters einen Führungsdraht zu verwenden. Dieser ist länger als der Katheter. Zur Verhinderung von Verletzungsgefahren haben Führungsdrähte eine geschmeidige Spitze. Der Vorteil des Führungsdrahtes besteht darin, daß er sich - da er steifer ist als der Katheter - zielsicherer als dieser im Gefäß vorschieben läßt und daß er den über ihn vorgeschobenen Katheter versteift und geführt zum Zielort bringt. Diese Praxis der Katheterverlegung wird "Seldinger-Technik" genannt, und sie wird sowohl bei kurzen als auch bei langen Kathetern angewendet. Es wird dabei im allgemeinen zunächst das Blutgefäß mit dem aus Punktionskanüle und aufgeschobenem Kurzkatheter bestehenden Besteck oder ausschließlich mit einer Punktionskanüle punktiert. Sodann wird durch die plazierte Punktionskanüle der Führungsdraht in das Gefäß eingeführt und es wird anschließend über den Führungsdraht der Katheter in das Gefäß vorgeschoben. Der Führungsdraht wird dann herausgezogen und verworfen. Die getrennt verwendeten Einzelteile sind für den Anwender unbequem, weil das Einfädeln bzw. Auffädeln der Teile zeitaufwendig ist und besondere Aufmerksamkeit verlangt.

Zur Vermeidung der Nachteile der Handhabung von Einzelteilen wurden diese zu einem Katheterisierungsbesteck zusammengebaut, das als Einheit handhabbar ist. Ein derartiges Katheterisierungsbesteck ist in EP-A-00 93 164 beschrieben. Dabei ist hinten (proximal) an den Kanülenansatz der mit dem Kurzkatheter zusammengesteckten Punktionskanüle ein langes Rohrteil angesetzt, das den Führungsdraht enthält, der mit Hilfe eines durch einen Schlitz in das Innere des Rohrteils ragenden Schiebers axial verschiebbar ist derart, daß sein vorderes Ende aus der Punktionskanüle vortritt. Dieses Katheterisierungsbesteck läßt sich infolge seiner beträchtlichen Länge und des Fehlens einer Griffvorrichtung schlecht handhaben, was sich nachteilig auf die Punktionssicherheit auswirkt.

Die Punktion erfolgt mit zurückgezogenem Führungsdraht, damit bei Punktionserfolg Blut in der Punktionskanüle zurückströmt und in den transparenten Kanülenansatz gelangt. Da das Besteck jedoch an diesem Kanülenansatz gehalten wird, verdecken die Finger ihn und der Blutaustritt wird nicht oder erst spät im Kanülenansatz entdeckt. Auch bei diesem Besteck ist ungünstig, daß bei der Gefäßpunktion nicht nur die Punktionskanüle, sondern auch der diese umgebende Katheter das Punktionsloch durchdringen muß. Der Hautdurchtritt erfolgt aufgrund des Hautwiderstandes ruckartig und die genaue Plazierung der Punktionskanülenspitze im Gefäß wird erschwert bzw. häufig unmöglich.

Eine gewisse Verbesserung der Ermittlung des Punktionserfolges wird mit einem Besteck nach US-A-48 94 052 erreicht, das insbesondere für die arterielle Punktion bestimmt ist. Der Katheter dieses Bestecks besteht aus durchsichtigem Kunststoffmaterial und in der durch den Katheter hindurchgeschobenen, nur mit dem Schliffabschnitt des Spitzenteils über den Katheter vorstehenden Punktionskanüle ist ein Führungsdraht verschiebbar vorgesehen. Die Punktionskanüle hat ein seitliches Loch, das bei anwendungsbereitem Katheterisierungsbesteck hinter dem Ende des Katheters liegt, d. h. von diesem umschlossen ist. Bei Punktionserfolg dringt Blut durch das Loch in einen Ringraum zwischen Punktionskanüle und transparentem Katheter und ist beim Aufsteigen in Richtung der Ansätze durch den Katheter sichtbar. Die Notwendigkeit der Belassung eines Ringraumes zum Blutdurchlaß bedingt eine Vergrösserung des Außendurchmessers des Katheters und erschwert damit die Punktion von Haut- und Gefäßwand, weil der dicke Katheter ein ruckartiges Vordringen bewirkt, das die genaue Plazierung der Punktionskanülenspitze im Gefäß in Frage stellt. Außerdem ist der Blutaustritt aus dem Loch nicht sofort erkennbar, weil dieser Teil im Gewebe steckt. Es muß das Aufsteigen von Blut gegen die Ansätze abgewartet werden.

Aus DE-U-89 14 941 ist ein Katheterisierungsbesteck bekannt, das außer einer Stahlkanüle und einem Kunststoffkapillar eine Dilatatorhülse aufweist. Die Dilatatorhülse wird auf die Stahlkanüle aufgeschoben und über sie wird das Kunststoffkapillar geschoben. Dieses Katheterisierungsbesteck dient dazu, großlumige Gefäßkatheter durch das Kunststoffkapillar in das Blutgefäß einzuführen. Zur Erzeugung eines hinreichend großlumigen Punktionsloches ist die kleinlumige Stahlkanüle von der relativ dicken Dilatatorhülse umgeben. Aus der Dilatatorhülse und dem darauf aufgeschobenen Kunststoffkapillar ragt die Stahlkanüle hervor. Bei der Punktion erreichen die Dilatatorhülse und das Kunststoffkapillar zunächst nur den Subcutanbereich während die dünne Stahlkanüle sogleich in den kritischen Bereich des Gefäßverlaufs vorgeschoben wird. Sobald durch Rückfluß von Blut der Punktionserfolg festgestellt ist, wird die Dilatatorhülse mit dem auf ihr sitzenden Kunststoffkapillar auf der Stahlkanüle vorgeschoben bis das distale Ende des Kunststoffkapillars sich im Blutgefäß befindet. Ein Führungsdraht ist hierbei nicht vorhanden, so daß der durch das Kunststoffkapillar einzuführende Katheter lediglich über die Länge des Kunststoffkapillars geführt wird. Das Einbringen des Katheters in den Körper mit Hilfe der Seldinger-Technik ist ausgeschlossen. Es können auch keine unterschiedlichen Methoden der Kathetereinführung benutzt werden.

Der Erfindung liegt die Aufgabe zugrunde, ein Katheterisierungsbesteck aus Stahlnadel, Kapillar und Führungsdraht zu schaffen, das eine Katheterverlegung mit hoher Genauigkeit nach unterschiedlichen Einführungsmethoden ermöglicht.

Die Lösung dieser Aufgabe erfolgt erfindungsgemäß mit den im Patentanspruch 1 angegebenen Merkmalen.

Die Punktion der Arterie bzw. Vene wird ausschließlich mit dem vom Kapillar freien, blanken Stahlteil der Stahlnadel durchgeführt, wodurch eine präzise Punktion des Gefäßes möglich ist. Das Kapillar auf der Stahlnadel durchtritt die Haut zunächst nicht. Infolge des Fehlens einer "Stufe" am Umfang der Stahlnadel geschieht der Hautdurchtritt praktisch ruckfrei und geradlinig, so daß die Stahlnadelspitze an der vom Anwender gewünschten Stelle plaziert wird. Entsprechend exakt wird der Führungsdraht zum Zielort geführt und wird der Katheter verlegt.

Mit dem erfindungsgemäßen Katheterisierungsbesteck erfolgt die Kathetereinführung nach zwei Methoden:
a) über die noch in der Arterie steckende Punktionskanüle zur besseren Führung durch Haut und Stichkanal, wobei der Führungsdraht die Führung im Lumen der Arterie übernimmt,
b) über den Führungsdraht bei vorher zurückgezogener Punktionskanüle.

Auf beide Methoden wirkt sich die gute Festhaltungsmöglichkeit des Bestecks durch die verbesserte Punktionsgenauigkeit mit Hilfe des blanken Punktionskanülenüberstandes günstig aus.

In weiterer vorteilhafter Ausgestaltung der Erfindung ist gemäß Anspruch 2 vorgesehen, daß in der Wand der Stahlnadel vor der Spitze des Katheters ein Loch ausgebildet ist. Bei hinter das Loch zurückgezogenem, als Lumenverschluß wirksamem Führungsdraht, oder nur in den Schliff zurückgezogenen, einen Ringraum zur Kanülenwand belassendem Führungsdraht wird die erfolgte Punktion des Gefäßes durch geringen Blutaustritt aus dem freiliegenden Stahlnadelloch sofort erkannt. Der Anwender verliert keine Zeit durch Abwarten des Blutaufstiegs bis zum Kanülenansatz. Falls kein Blutaustritt am Stahlnadelloch erfolgt, kann er die Punktion unverzüglich wiederholen. Die Punktionssicherheit wird erhöht. Auch werden die hygienischen Bedingungen verbessert, weil störender Blutaustritt am anwenderseitigen Ende weitestgehend vermieden wird. Durch Verminderung der Kontaminationsgefahr mit Blut werden die Arbeitsbedingungen verbessert. Das Loch in der Stahlnadel kann gebohrt, geschliffen oder gestanzt sein. Es befindet sich bevorzugt in unmittelbarer Nähe der Spitze des Katheters, damit der blanke Stahlnadelabschnitt über im wesentlichen seine ganze Länge als Punktionselement ausgenutzt werden kann. Die Lochgröße soll so bemessen sein, daß der Blutaustritt wahrnehmbar ist, ein Herausrinnen von größeren Blutmengen jedoch vermieden wird.

Das Katheterisierungsbesteck nach dem Anspruch 1 wird erfindungsgemäß zusätzlich so verbessert, daß nach erfolgter Plazierung des Katheters, insbesondere in einer Arterie, ein Blutrückfluß verhindert, jedoch ein einfacher Anschluß eines Übertragungsgerätes möglich ist.

Zu diesem Zweck ist gemäß Anspruch 3 in dem Katheteransatz ein Ventilkörper mit einem selbstschließenden Durchlaß angeordnet. Der Durchlaß in dem Ventilkörper ermöglicht den ausstanzungsfreien Durchtritt der Punktionskanüle mit innenliegendem Führungsdraht. Wenn nach erfolgreicher Punktion die Punktionskanüle und der Führungsdraht aus dem Katheter herausgezogen worden sind, schließt sich der Durchlaß des Ventilkörpers und es kommt nicht zum Blutrückstrom aus dem Katheteransatz. Der Katheter kann in Ruhe auf der Haut des Patienten befestigt werden, z. B. mit Hilfe von Flügeln festgenäht werden, ohne daß die Arterie abgedrückt werden muß.

Vorteilhafterweise besteht der Ventilkörper aus einer Elastomerscheibe mit mindestens einem Schlitz, und es ist vorgesehen, daß ein mit dem Katheteransatz verbindbares Anschlußstück einer Schlauchleitung einen koaxialen rohrförmigen Offenhalter trägt, der den Schlitz durchdringt. Das direkt an den Katheteransatz anschließbare Anschlußstück bewirkt über den rohrförmigen Offenhalter eine Öffnung des Ventilkörpers im Katheteransatz und über die Schlauchleitung ist eine flexible Verbindung mit einem Übertragungsgerät möglich. Hierdurch wird die Gefahr der Dislokation der Arterie vermieden, weil die Schlauchleitung sich so legen und handhaben läßt, daß kein Zug auf den im Gefäß befindlichen Katheter ausgeübt wird. Eine weitere Handhabungsverbesserung ergibt sich dadurch, daß die Schlauchleitung an dem dem Anschlußstück abgewandten Ende einen Hahn, vorzugsweise einen Dreiwegehahn, aufweist, der nach Öffnung des Durchlasses in dem Ventilkörper eine Flüssigkeits- oder Druckübertragung ermöglicht.

In der Zeichnung ist ein Ausführungsbeispiel der Erfindung schematisch dargestellt. Es zeigen:

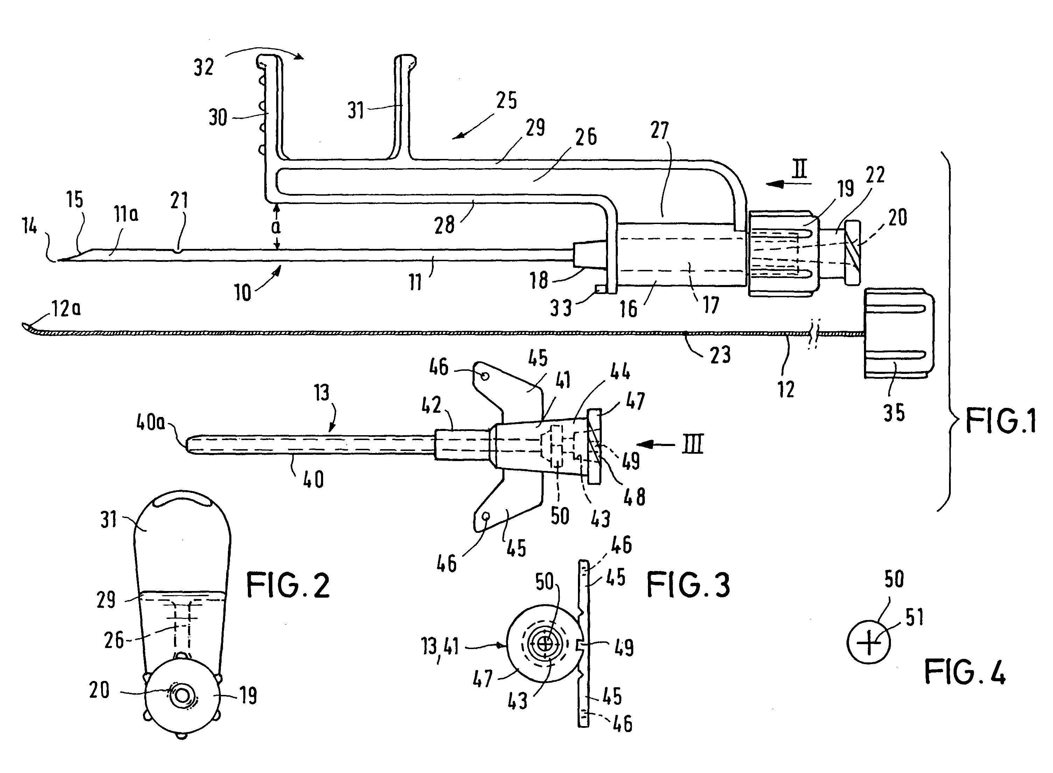

- Fig. 1: drei Bestandteile eines Katheterisierungsbestecks, die im Anwendungszustand koaxial zusammengesteckt sind,
- Fig. 2: eine Ansicht in Richtung des Pfeils II in Fig. 1,
- Fig. 3: eine Ansicht in Richtung des Pfeils III in Fig. 1,
- Fig. 4: eine Draufsicht auf eine Ventilscheibe für den Katheteransatz,
- Fig. 5: eine Draufsicht auf das anwendungsbereite Katheterisierungsbesteck,
- Fig. 6: das vordere Ende des Katheterisierungsbestecks in vorgeschrittenem Anwendungsstadium, und
- Fig. 7: den im Gefäß verlegten und auf der Haut festgenähten Katheter kurz vor der Öffnung des Ventils im Katheteransatz durch einen Offenhalter an einer Anschlußvorrichtung.

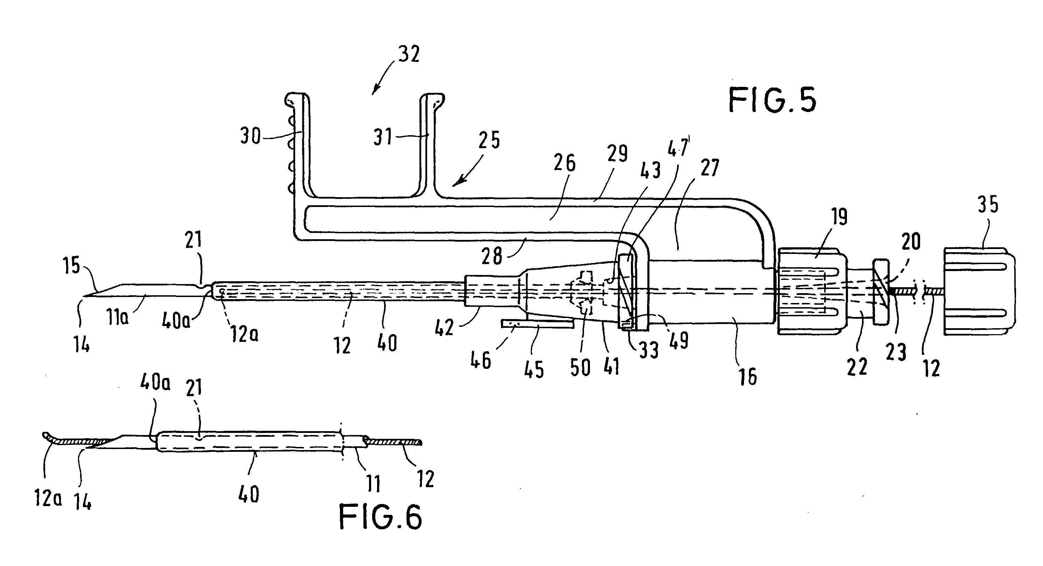

Ein Katheterisierungsbesteck, das sich besonders zur Katheterisierung von Arterien eignet, besteht im wesentlichen aus drei Teilen, nämlich einer Punktionskanüle 10, einem Führungsdraht 12 und einem Katheter 13, die sich - wie in Fig. 5 gezeigt - koaxial zusammenstecken lassen. Die Punktionskanüle 10 weist eine hohle Stahlnadel 11 auf, deren vorderes (distales) Ende 15 spitz und scharf angeschliffen ist und an deren hinterem (proximalen) Ende ein Kanülenansatz 16 befestigt ist, der einen durchgehenden Kanal 17 enthält. Ein Außenkonus 18 am Übergang zwischen der Stahlnadel 11 und dem Kanülenansatz 16 dient als Steckelement zur Verbindung mit einem Innenkonus eines Katheteransatzes des Katheters 13. Eine Blutrückhaltekappe 19 mit einem koaxialen engen Durchlaß 20 zur Führung des Führungsdrahtes 12 schließt den Kanülenansatz 16 am hinteren Ende ab. Die Blutrückhaltekappe 19 trägt am hinteren Ende einen koaxialen Kupplungsstutzen 22 mit einem Verriegelungselement für einen Anschluß. In einem Abstand von etwa 18 mm hinter der Spitze 14 der Stahlnadel 11 ist in ihrer Wand ein kleines Loch 21 ausgebildet, das gebohrt, geschliffen oder gestanzt sein kann und das von außen bis in das Lumen der Stahlnadel 11 durchgeht.

Mit dem Kanülenansatz 16 der Punktionskanüle 10 ist eine Griffvorrichtung 25 verbunden. Vorzugsweise sind der Kanülenansatz 16 und die Griffvorrichtung 25 aus Kunststoff gefertigt und einstückig hergestellt. Die Griffvorrichtung 25 weist einen Arm 26 auf, der parallel zur Stahlnadel 11 mit Abstand neben dieser verläuft. Das hintere (proximale) Ende des Armes 26 schließt sich über einen rechtwinklig abgehenden Schenkel 27 an einen rohrförmigen Hauptkörper des Kanülenansatzes 16 an. Vorzugsweise ist die Breite des Schenkels 27 im wesentlichen gleich der Länge des Hauptkörpers. Seine Erstreckung in Querrichtung bestimmt den Abstand a zwischen der Stahlnadel 11 und dem ihr nächstliegenden Rand 28 des Armes 26. Der Hauptbereich des Armes 26 ist plattenartig flach während seine beiden Längsränder 28 und 29 zur Stabilisierung wulstartig verstärkt sind. An dem vorderen (distalen) Ende des Armes 26 sind zwei Griffplatten 30, 31 angeformt, die in gleicher Flucht von dem Längsrand 29 rechtwinklig zur Seite abstehen. Die beiden Griffplatten 30, 31 bilden ein Griffstück 32, das zwischen Daumen und Zeigefinger erfaßt werden kann. Die Länge des Armes 26 und die Anbringung des Griffstückes 32 an diesem sind so gewählt, daß das Griffstück 32 sich im Mittelbereich der Stahlnadel 11 befindet. Ein bevorzugter Abstand zwischen der Spitze 14 und der Griffplatte 30 beträgt etwa 45 mm.

Durch den Kanülenansatz 16 und die Stahlnadel 11 ist der Führungsdraht 12 hindurchführbar, dessen Durchmesser dem Lumendurchmesser angepaßt ist und der an seinem proximalen Ende ein Kopfstück 35 trägt, das als Verschlußkappe mit Innengewinde ausgebildet und mit einem Gewindeabschnitt des Kupplungsstutzens 22 verriegelbar ist. Der Führungsdraht 12 kann als eng gewickelter Stahldraht mit flexibler Spitze 12a gestaltet sein. Das Katheterisierungsbesteck ist jedoch auch mit einem Führungsdraht funktionsfähig, dessen hinteres Ende als starrer Draht ausgeführt ist und der nur im vorderen Teil als schraubenförmig gewikkelter flexibler Strangkörper ausgebildet ist. Wenn der Führungsdraht 12 bis zum Anschlag des Kopfstückes 35 gegen die Blutrückhaltekappe 19 in die Punktionskanüle 10 eingeschoben und durch eine Drehbewegung mit dem Kupplungsstutzen 22 der Blutrückhaltekappe 19 verriegelt ist, überragt sein vorderes Ende die Spitze 14 der Stahlnadel 11 um ein zur Durchführung der Führungsfunktion im jeweiligen Anwendungsfalle benötigtes Stück, das etwa 20 mm bis 50 mm lang sein kann.

Der Katheter 13 ist ein Kurzkatheter aus flexiblem Kunststoff, der 45 mm bis 50 mm lang sein kann und aus einem Kapillar 40 besteht, an dessen hinterem Ende ein Katheteransatz 41 befestigt ist. Der Katheteransatz 41 ist ein Rohrstück, das am vorderen (distalen) Ende einen flexiblen Knickschutz 42 für das Kapillar 40 aufweist und am hinteren (proximalen) Ende einen Innenkonus 43 zur Aufnahme des Außenkonus 18 der Punktionskanüle 10 sowie eine Kammer 44 zur Unterbringung einer Ventilvorrichtung enthält. An der Außenseite des Katheteransatzes 41 sind zwei nach entgegengesetzten Seiten gerichtete Fixierflügel 45 angebracht, die Löcher 46 zum Festnähen auf der Haut des Patienten aufweisen.

Die hintere Öffnung des Katheteransatzes 41 ist von einem Ringflansch 47 umgeben, der Kupplungselemente 48, z. B. Gewindeteile, zur Verbindung mit einem Konnektor aufweist, der weiterführende Leitungen an den Katheter 13 anschließt. In dem Ringflansch 47 ist eine radiale Kerbe 49 ausgebildet, in die ein axialer Zapfen 33 des Kanülenansatzes 16 als Drehsicherung eingreift. In der Kammer 44 sitzt eine Elastomerscheibe 50, die von einem Schlitz oder Kreuzschlitz 51 durchsetzt ist, dessen Lippen dicht aneinander liegen und den Kanal des Katheters 13 nach hinten absperren.

Für den Transport in steriler Verpackung ist der Katheter 13 auf die Punktionskanüle 10 aufgesteckt und durch Zusammengriff des Außenkonus 18 mit dem Innenkonus 43 an dieser festgelegt. In diesem Zusammenbau-Zustand überragt die Stahlnadel 11 mit einem Abschnitt 11a die Spitze 40a des Kapillars 40 um vorzugsweise 20 mm und das Loch 21 liegt frei vor der Kapillarspitze 40a (Fig. 5). Der Führungsdraht 12 ist durch die Punktionskanüle 10 hindurchgeschoben und ragt aus dem Schliffende 15 der Stahlnadel 11 ein Stück vor. Eine nicht gezeichnete Schutzkappe deckt die in den Patienten einzuführenden Punktionselemente ab.

Zur Punktion wird der Führungsdraht 12 zurückgezogen bis eine auf dem Führungsdraht 12 angebrachte Markierung 23 mit der Hinterkante der Blutrückhaltekappe 19 längengleich übereinstimmt und seine Spitze 12a hinter dem Loch 21 der Stahlnadel 11 liegt (Fig. 5). Das Katheterisierungsbesteck wird an dem abgewinkelten Griffstück 32 erfaßt und es wird die Stahlnadel 11 ausschließlich mit ihrem von dem Kapillar 40 freien blanken Teil 11a in ein Blutgefäß eingestochen. Das Loch 21 bleibt außerhalb der Einstichstelle sichtbar. Die erfolgreiche Punktion ist durch Blutaustritt aus dem Loch 21 unverzüglich erkennbar. Sodann wird der Führungsdraht 12 in das Gefäß vorgeschoben und sein Kopfstück 35 mit der am Kanülenansatz 16 befestigten Blutrückhaltekappe 19 verriegelt. Der Vorschub des Führungsdrahtes 12 verschließt das Loch 21 und beendet den Blutaustritt. Auch ein Blutrückstrom wird von dem Führungsdraht 12 weitestgehend unterbunden, da dieser infolge seines dem Lumen der Stahlnadel 11 angepaßten Durchmessers als Lumenverschluß wirksam ist. Das Kapillar 40 kann über die aus dem Punktionsloch etwas zurückgezogene Stahlnadel 11 und den Führungsdraht 12 in das Gefäß geschoben werden. Anschließend werden die Stahlnadel 11 mit dem innenliegenden Führungsdraht 12 in toto aus dem Katheter 13 herausgezogen. Der Kreuzschlitz 51 der Elastomerscheibe 50 im Katheteransatz 41, der sich zum Durchlaß der Stahlnadel 11 ohne weiteres geöffnet und mit seinen vier Lappen an den Umfang der Stahlnadel 11 abdichtend angelegt hat, schließt sich nach Herausziehen von Stahlnadel 11 und Führungsdraht 12 abdichtend, so daß es nicht zum Blutrückstrom aus dem Katheter 13 kommt. Der Katheter 13 wird mittels der Fixierflügel 45 auf der Haut des Patienten festgenäht, so daß die Spitze 40a des Kapillars 40 in der korrekten Position innerhalb des Gefäßes festgelegt ist.

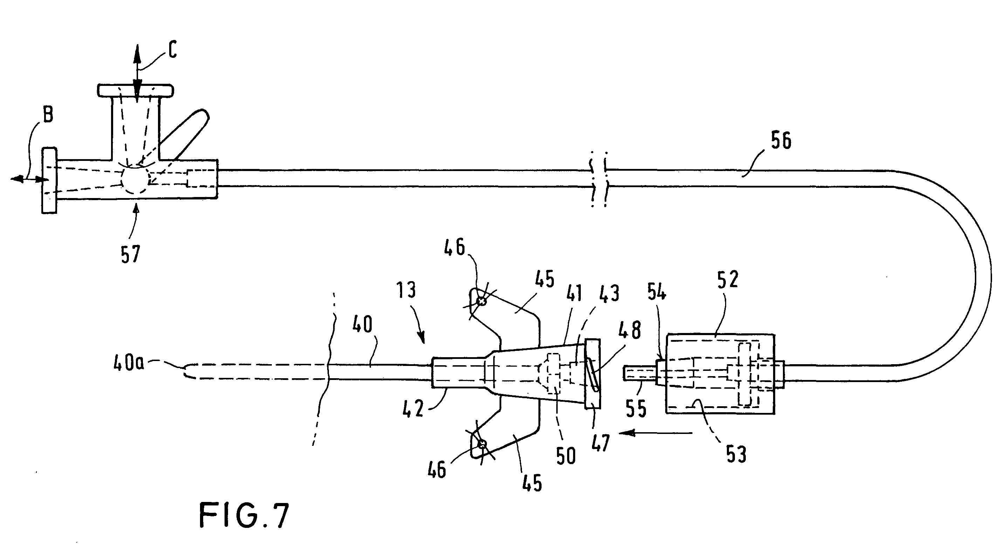

Zum Anschluß eines Übertragungsgerätes an den Katheter 13 dient eine flexible Schlauchleitung 56, deren eines Ende ein Gehäuse eines Dreiwegehahns 57 mit Ein- bzw. Ausgängen B und C trägt (Fig. 7). Am anderen Ende der Schlauchleitung 56 befindet sich ein Anschlußstück 52, das ein Innengewinde 53 zum Aufschrauben auf die Kupplungselemente 48 des Ringflansches 47 des Katheteransatzes 41 aufweist. Ein koaxialer Außenkonus 54, der in den Innenkonus 43 des Katheteransatzes 41 paßt, wird von einem rohrförmigen Offenhalter 55 nach außen fortgesetzt. Der Offenhalter 55 mit sehr geringen Abmessungen durchdringt im Konnektierungszustand der Teile 41 und 52 den Kreuzschlitz 51 der Elastomerscheibe 50 und hält den Ventilkörper 50 zum Durchströmen einer Flüssigkeit offen. Durch Umschalten des Dreiwegehahns 57 werden die Ein- bzw. Ausgänge B oder C geöffnet bzw. geschlossen.

## Patentansprüche

1. Katheterisierungsbesteck für die Katheterverlegung in einem Blutgefäß, bestehend aus einer Punktionskanüle (10) mit einer Stahlnadel (11) und einem Kanülenansatz (16),
einem die Stahlnadel (11) umgebenden Kapillar (40) mit einem Katheteransatz (41),
und einem Führungsdraht (12), der in dem Lumen der Stahlnadel (11) axial verschiebbar ist,
**dadurch gekennzeichnet**, daß im Zusammenbau-Zustand die Stahlnadel (11) mit einem Abschnitt (11a) die Spitze (40a) des Kapillars (40) mm 15 mm bis 25 mm, vorzugsweise mm 20 mm, überragt.

2. Katheterisierungsbesteck nach Anspruch 1, dadurch gekennzeichnet, daß in der Wand der Stahlnadel (11) distal von der Spitze (40a) des Kapillars (40) ein Loch (21) ausgebildet ist.

3. Katheterisierungsbesteck nach Anspruch 1 oder 2, dadurch gekennzeichnet, daß in dem Katheteransatz (41) ein Ventilkörper mit einem selbstschließenden Durchlaß angeordnet ist.

4. Katheterisierungsbesteck nach Anspruch 3, dadurch gekennzeichnet, daß der Ventilkörper aus einer Elastomerscheibe (50) mit mindestens einem Schlitz (51) besteht und daß ein mit dem Katheteransatz (41) verbindbares Anschlußstück (52) einer Schlauchleitung (50) einen koaxialen rohrförmigen Offenhalter (55) trägt, der den Schlitz (51) der Elastomerscheibe (50) durchdringt.

5. Katheterisierungsbesteck nach Anspruch 4, dadurch gekennzeichnet, daß die Schlauchleitung (50) an dem dem Anschlußstück (52) abgewandten Ende einen Hahn (57), vorzugsweise einen Dreiwegehahn, aufweist.

## Claims

1. Catheterisation set for placing a catheter in a blood vessel, comprising a puncturing cannula (10) with a steel needle (11) and a cannula hub (16),
- a capillary (40) having a catheter hub (41) and surrounding the steel needle (11),
- and a guide wire (12) axially displaceable within the lumen of the steel cannula (11),
characterized in that
in the assembled state, a portion (11a) of the steel needle (11) protrudes beyond the tip (40a) of the capillary (40) by 15 mm to 25 mm, preferably by 20 mm.

2. The catheterisation set of claim 1, characterized in that distal of the tip (40a) of the capillary (40) the wall of the steel needle (11) is provided with a hole (21).

3. The catheterisation set of claim 1 or 2, characterized in that a valve body with a self-closing passage is arranged in the catheter hub (41).

4. The catheterisation set of claim 3, characterized in that the valve body is an elastomer disc (50) with at least one slit (51), and that a connector piece (52) of a hose conduit (56) adapted to be connected to the catheter hub (41) bears a coaxial tubular means (55) for keeping said connector piece open, which means penetrates the slit (51) in the elastomer disc (50).

5. The catheterisation set of claim 4, characterized in that the hose conduit (56) has a tap (57), preferably a three-way tap, at the end averted from the connector piece (52).

## Revendications

1. Set de cathétérisme pour la pose d'un cathéter dans un vaisseau sanguin, constitué d'une canule de ponction (10) comprenant une aiguille d'acier (11) et un embout de canule (16),
d'un capillaire (40) entourant l'aiguille d'acier (11) et comportant un embout de cathéter (41),
et d'un fil de guidage (12) qui peut coulisser axialement dans la lumière de l'aiguille d'acier (11),
caractérisé en ce que dans l'état assemblé, l'aiguille d'acier (11) dépasse, avec un tronçon (11a), de la pointe (40a) du capillaire (40), sur 15 mm à 25 mm, de préférence sur 20 mm.

2. Set de cathétérisme selon la revendication 1, caractérisé en ce que dans la paroi de l'aiguille d'acier (11), un trou (21) est formé en position distale par rapport à la pointe (40a) du capillaire (40).

3. Set de cathétérisme selon la revendication 1 ou 2, caractérisé en ce que dans l'embout de cathéter (41) est disposé un corps d'obturation de soupape comportant une ouverture de passage à auto-fermeture.

4. Set de cathétérisme selon la revendication 3, caractérisé en ce que le corps d'obturation de soupape est constitué par un disque en élastomère (50) comportant au moins une fente (51) et en ce qu'une pièce de raccordement (52) d'un conduit tubulaire souple (56), qui peut être reliée à l'embout de cathéter (41), porte un élément de maintien d'ouverture (55) tubulaire coaxial, qui traverse la fente (51) du disque en élastomère (50).

5. Set de cathétérisme selon la revendication 4, caractérisé en ce que le conduit tubulaire souple (56) comprend, à l'extrémité située à l'opposé de la pièce de raccordement (52), un robinet (57), de préférence un robinet à trois voies.
